# EUROPEAN PATENT APPLICATION

(11) **EP 0 697 583 A1**
(43) Date of publication of application: **21.02.1996**
(21) Application number: 94202344.1
(22) Date of filing: 18.08.1994
(51) Int. Cl.: G01F 23/14, G01F 23/296, G01F 23/284, G01F 23/292, G01N 29/18

(54) **Liquid/foam mixture analysis**

(71) Applicant: THE SECRETARY OF STATE FOR DEFENCE IN HER BRITANNIC MAJESTY'S GOVERNMENT OF THE UNITED KINGDOM OF GREAT BRITAIN AND NORTHERN, London SW1A 2HB (GB)
(72) Inventor: Bolland, Michael David, Reading, Berkshire RG3 3PB (GB); Thompson, Douglas, Oakley, Hampshire RG23 7BW (GB)
(74) Representative: Lockwood, Peter Brian

(57) **Abstract**

A method of analysing the physical composition of a liquid/ foam mixture (20, 21) includes the steps of;
positioning a pressure sensor (12) adjacent a base of a volume of the mixture;
positioning a transmitter and a receiver combination (13) above a boundary surface (22) of the mixture, the transmitter being adapted to transmit a signal which will be reflected from the surface (22) and detected by the receiver;
passing signals from the pressure transducer (12) and from the transmitter and receiver combination (13) to an analyser (15); and
comparing the signals with a calibration to provide an analysis of the mixture combination.

Apparatus is provided for carrying out this method.

## Description

The present invention is concerned with apparatus and methods for use in the analysis of liquid/ foam mixtures.

There are many instances where a liquid is caused to intermix with a gas to form a foam. This may be deliberate as, for example, when foam is being prepared as a fire extinguisher or in some chemical processes, or accidental as, for example, in the hold of an oil tanker in heavy seas. In such instances it is frequently desirable to know what proportion of a mixture is pure liquid and what proportion is foam. Knowledge of the various proportions may be of assistance in optimising desired characteristics of the mixture, or may provide information regarding the integrity of a container holding the mixture.

According to one aspect of the present invention a method of analysing the physical composition of a liquid/ foam mixture is characterised in that it includes the steps of;
positioning a pressure sensor adjacent a base of a volume of the mixture;
positioning a transmitter and a receiver combination above a boundary surface of the mixture, the transmitter being adapted to transmit a signal which will be reflected from the surface and detected by the receiver;
passing signals from the pressure transducer and from the transmitter and receiver combination to an analyser; and
comparing the signals with a calibration to provide an analysis of the mixture combination.

In the simplest form of calibration the pressure transducer can be placed in a container containing a known head of pure liquid and its reading noted, the liquid then being replaced with a foam of known density, preferably corresponding to the volume of liquid providing the known head, and the reading from the transmitter and receiver combination positioned above the surface of the foam noted. This form of calibration is particularly of use in cases where analysis of the composition of a fixed volume of liquid, for example the contents of a compartment of an oil tanker, is required. Other forms of calibration might be required when the volume of liquid being analysed is variable, and might involve calibration of the pressure sensor with various heads of liquid and of the transmitter and receiver combination with various foam surface levels, various foam consistencies, or both.

The surface reflecting the signal will usually be the foam surface, but as an alternative, or as an addition, the surface forming the interface between the foam and the liquid might be used. When measurements requiring reflection from both the interface and the foam surfaces are required it might be necessary to adjust the signal according the surface being used at any particular time.

The transmitter and receiver combination might of course comprise a single unit such as, for example, an ultrasonic sensor. Other signal systems might be, for example, laser, infra red, radar, microwave or visible light. The type of transmitter and receiver combination for a particular location will usually depend on various considerations such as cost, safety and required accuracy of the results.

One or more additional pressure transducers might be installed within the volume to give additional information.

According to another aspect of the invention apparatus for analysing the composition of a liquid/foam mixture includes a pressure transducer adapted to be positioned adjacent a bottom of a container and a transmitter and receiver combination adapted to be positioned above a surface in the container, the transmitter being adjusted to transmit a signal which is reflected from the surface and detected by the receiver, the pressure transducer and the transmitter and receiver combination being connected to provide signals to analysis equipment wherein they are combined with a calibration to provide an analysis of the mixture.

The surface will usually be the foam surface, but may alternatively or additionally be the surface forming the interface between the liquid and the foam.

The transmitter and receiver combination may use ultrasound signals, radar signals, a laser, infra-red, visible light or any other suitable signal, and the combination may in practice consist of a single unit such as an ultrasound sensor. The combination may be such that characteristics of the signal used, such as the wavelength thereof, are adjustable.

The analysis equipment may be positioned adjacent to or remote from the container, and might provide a direct reading of the analysis or transmit the results, for example by radio signal, to a remote location.

One embodiment of the invention will now be described, by way of example only, with reference to the accompanying diagrammatic drawings, of which;
Figure 1 shows an elevation, partly in section, of an oil tanker in which is installed apparatus according to the invention for use according to the method of the invention, and
Figure 2 is an elevation, in section, of a detail of the tanker shown in Figure 1.

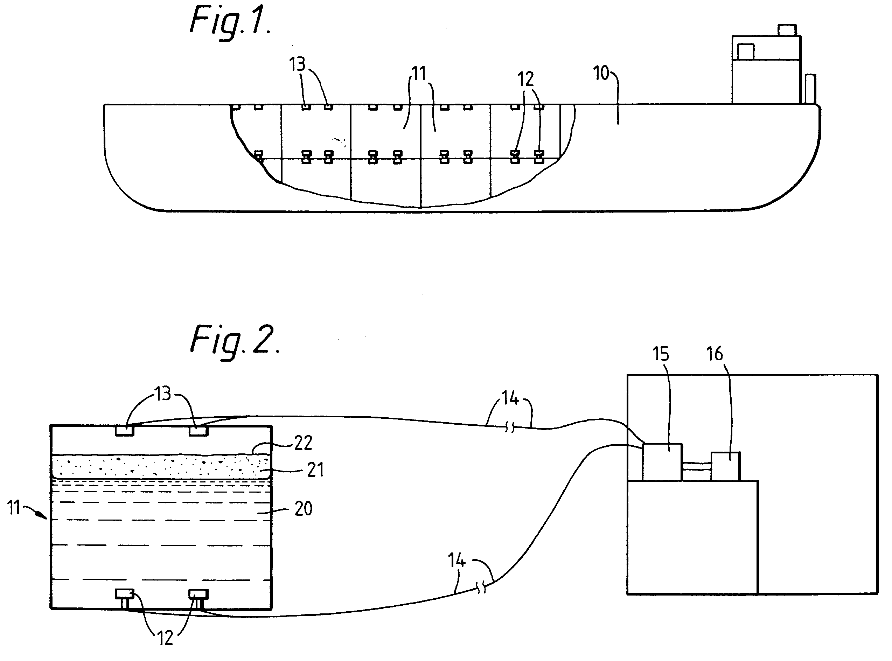

An oil tanker 10 (Figure 1) contains a number of containers in the form of individual compartments such as those shown at 11. Adjacent the bottom of each compartment 11 (see especially Figure 2) is at least one pressure sensor 12, and adjacent the top of each compartment 11 is at least one transmitter and receiver combination in the form of an ultrasonic sensor 13.

For each compartment 11 associated pressure sensors 12 and ultrasonic sensors 13 are connected, either directly by cables 14 as shown in Figure 2 or indirectly, for example by radio transmission, to analysis equipment 15 providing a read-out to a display 16, the analysis equipment and display being positioned in a ships control room.

Prior to installation in the compartments 11 the pressure transducers 12 are calibrated, for example by placing in test chambers corresponding to the compartments 11 and containing an appropriate volume of liquid, and the ultrasonic sensors 13 are calibrated, for example by placing in the test chambers containing foam of known consistency, the outputs respectively of the transducers 12 and of the sensors 13 being noted.

In use signals from each pressure transducer 12, giving the pressure adjacent the base of a compartment 11, and from each ultrasonic sensor 13, representative of a distance from a boundary surface 22 (Figure 2) are passed to the analysis equipment 15 where they are compared with the calibration to provide a reading to the display 16. In calm seas and after settling of a load of oil the contents of each compartment can be expected to be entirely liquid 20 (Figure 2), and any loss of pressure shown by a pressure transducer 12 will be an indication of a leak from the tank in which it is installed. In heavy seas, however, the contents of each compartment 11 will be agitated and will turn partly to foam 21. By use of the invention the proportions of liquid 20 and of foam 21 within a compartment can be determined and using the calibration it can be seen whether there is a loss of contents. The ratio between liquid and foam may also have implications regarding the inflammability level within a compartment 11 and a knowledge of this ratio will therefore allow precautions to be taken when required.

It will be realised that many variations of the above described embodiment are possible within the scope of the invention. Whilst a single pressure transducer 12 and a single ultrasonic sensor 13 within each compartment 11 may suffice, in general it is preferable to have a plurality, to allow for failures. It will usually be advisable to have each pressure transducer 12 positioned some distance above a compartment floor, to avoid spurious signals as a result of sludge. It will also be realised that many other types of transmitter and receiver, other than ultrasonic sensors, may be used. Examples are lasers, radar, infra-red and visible light.

In some instances it might be appropriate to use the surface forming the interface between the liquid and foam layers as the reflecting surface as an addition to or as an alternative to the foam surface. In such instances it might be necessary to have the sensor 13 such that characteristics, such as wavelength, of the signal can be adjusted.

In addition to their main purpose, either of the pressure transducer 12 or sensor 13 can be used to measure liquid or foam height in a container, for example during a filling process.

It will also be realised that whilst the apparatus and method according to the invention have been described with reference to an oil tanker there are many other uses for the invention- for example in the chemical industry where the relationship between liquid and foam may affect the speed of reactions, may have safety implications, or may have importance in some other way. Another important use is in the preparation or testing of fire-fighting foam, where the presence of liquid at the base of the foam may indicate a deterioration in the quality of the foam. In some uses of the invention the transducers 12 and sensors 13 might be adapted to provide readings adjacent to the containers with which they are being used, in others it may be more appropriate to have remotely situated facilities, transmission of information being made by cables, radio signals, or other means which will be obvious to those skilled in the art.

Methods and apparatus according to the invention can be used in any situation where the presence of foam is possible and where measures of liquid height, pressure, rate of filling, leakage, or other parameters are required.

## Claims

1. A method of analysing the physical composition of a liquid/ foam mixture (20,21) characterised in that it includes the steps of;
positioning a pressure sensor (12) adjacent a base of a volume of the mixture;
positioning a transmitter and a receiver combination (13) above a boundary surface (22) of the mixture, the transmitter being adapted to transmit a signal which will be reflected from the surface (22) and detected by the receiver;
passing signals from the pressure transducer (12) and from the transmitter and receiver combination (13) to an analyser (15); and
comparing the signals with a calibration to provide an analysis of the mixture combination.

2. A method as claimed in Claim 1 characterised in that the calibration is carried out by placing the pressure transducer (12) in a container containing a known head of pure liquid (20) and noting the reading thereof, and by then replacing the liquid with a foam (21) of known consistency and noting the reading from the transmitter and receiver combination (13) positioned above the surface of the foam.

3. A method as claimed in Claim 2 characterised in that the foam (21) corresponds to the volume of liquid providing the known head.

4. A method as claimed in Claim 1 characterised in that the calibration is carried out using various heads of liquid, and various foam surface levels, various foam consistencies, or both for calibration respectively of the pressure transducer and of the transmitter and receiver combination.

5. A method as claimed in any one of Claims 1 to 4 characterised in that the foam surface is used as a surface (22) to reflect the signal.

6. A method as claimed in any one of Claims 1 to 5 characterised in that the surface forming the interface between the foam (21) and the liquid (20) is used as a surface (22) to reflect the signal.

7. A method as claimed in any one of Claims 1 to 6 characterised in that the transmitter and receiver combination (13) comprise a single unit.

8. A method as claimed in any one of Claims 1 to 7 characterised in that the transmitter and receiver unit (13) operates using an ultrasonic signal.

9. A method as claimed in any one of Claims 1 to 7 characterised in that the transmitter and receiver unit (13) operates using a radar signal.

10. A method as claimed in any one of Claims 1 to 7 characterised in that the transmitter and receiver unit (13) operates using a laser signal.

11. A method as claimed in any one of Claims 1 to 7 characterised in that the transmitter and receiver unit (13) operates using an infra-red signal.

12. A method as claimed in any one of Claims 1 to 7 characterised in that the transmitter and receiver unit (13) operates using a visible light signal.

13. A method as claimed in any one of Claims 1 to 12 characterised in that at least one additional pressure transducer (12) is included within the volume.

14. Apparatus for analysing the composition of a liquid/foam mixture (20, 21) according to any one of Claims 1 to 14 characterised in that it includes a pressure transducer (12) adapted to be positioned adjacent a bottom of a container and a transmitter and receiver combination (13) adapted to be positioned above a surface (22) in the container, the transmitter being adjusted to transmit a signal which is reflected from the surface (22) and detected by the receiver, the pressure transducer (12) and the transmitter and receiver combination (13) being connected to provide signals to analysis equipment (15) wherein they are combined with a calibration to provide an analysis of the mixture.
